Europäisches Patentamt

European Patent Office

Office européen des brevets

(19)

(11) Publication number: **0 267 556 B1**

# EUROPEAN PATENT SPECIFICATION

(12)

(45) Date of publication of patent specification: 20.02.91

(21) Application number: 87116423.2

(22) Date of filing: 06.11.87

(51) Int. Cl.⁵: **C07C 47/22**, C07C 45/35, C07C 45/37, C07C 51/25, C07C 51/235, C07C 57/04, B01J 23/88, B01J 27/192, B01J 27/02, B01J 27/132

(54) **Process for production of methacrolein and methacrylic acid.**

(30) Priority: 11.11.86 JP 266550/86

(43) Date of publication of application:
18.05.88 Bulletin 88/20

(45) Publication of the grant of the patent:
20.02.91 Bulletin 91/08

(84) Designated Contracting States:
BE DE FR GB NL

(56) References cited:
EP-A- 0 102 641
FR-A- 2 354 812
FR-A- 2 435 456

PATENT ABSTRACTS OF JAPAN, vol. 10, no. 172 (C-354)[2228], 18th June 1986;

(73) Proprietor: MITSUBISHI RAYON CO., LTD.
3-19, Kyobashi 2-chome Chuo-Ku
Tokyo 104(JP)

(72) Inventor: Kuroda, Tooru
c/o MITSUBISHI RAYON COMPANY, LTD.,
20-1 Miyukicho
Ootake-shi Hiroshima 739-06(JP)
Inventor: Oh-Kita, Motomu
c/o MITSUBISHI RAYON COMPANY, LTD.,
20-1 Miyukicho
Ootake-shi Hiroshima 739-06(JP)
Inventor: Kato, Masaaki
c/o MITSUBISHI RAYON COMPANY, LTD.,
20-1 Miyukicho
Ootake-shi Hiroshima 739-06(JP)

(74) Representative: Hansen, Bernd, Dr.rer.nat. et al
Hoffmann, Eitle & Partner Patentanwälte Arabellastrasse 4 Postfach 81 04 20
D-8000 München 81(DE)

**Description**

BACKGROUND OF THE INVENTION

This invention relates to a process for production of methacrolein and methacrylic acid by gas phase catalytic oxidation of isobutylene or tert-butanol with molecular oxygen.

Many proposals have been made for catalysts used in production of methacrolein and methacrylic acid by gas phase catalytic oxidation of isobutylene or tert-butanol at high temperatures. However, from an industrial viewpoint, the catalysts used in this reaction have been demanded to be improved in catalytic efficiencies such as catalytic activity, selectivity for methacrolein and methacrylic acid, life of catalyst, etc.

U.S. Patent No. 4,537,874 discloses MoWBiFe[Ni, Co] alkali metal catalysts, but makes no mention of Cr. Besides, feature of the patent resides in subjecting Bi compound and W compound to previous calcination treatment at 600 - 900°C and preparation of catalysts is complicated.

SUMMARY OF THE INVENTION

This invention is a process for producing methacrolein and methacrylic acid, characterized by gas phase catalytic oxidation of isobutylene or tert-butanol with a molecular oxygen-containing gas in the presence of a catalyst having a composition represented by the following formula:

$$Mo_aW_bBi_cFe_dSb_eCr_fSi_gA_hX_iY_jZ_kO_\ell$$

wherein Mo, W, Bi, Fe, Sb, Cr, Si and O represent molybdenum, tungsten, bismuth, iron, antimony, chromium, silicon and oxygen, respectively, A represents at least one element selected from the group consisting of nickel and cobalt, X represents at least one element selected from the group consisting of potassium, rubidium, cesium and thallium, Y represents at least one element selected from the group consisting of magnesium, zinc, manganese, cadmium, lead and barium, Z represents at least one element selected from the group consisting of phosphorus, boron, sulfur, chlorine, cerium, tin and titanium and a, b, c, d, e, f, g, h, i, j, k and $\ell$ each represents atomic ratio of each element and when a = 12, b = 0.01-2, preferably 0.03-1, c = 0.01-3, preferably 0.1-2, d = 0.5-4, preferably 1-3.5, e = 0.01-3, preferably 0.1-2, f = 0.01-3, preferably 0.03-1, g = 1-20, preferably 3-18, h = 1-12, preferably 3-10, i = 0.01-2, preferably 0.05-1, j = 0.01-5, preferably 0.1-4 and k = 0-3, preferably 0-1 and $\ell$ is the number of oxygen atom necessary to satisfy the valency of each of said component.

DESCRIPTION OF THE INVENTION

As starting materials for said elements in preparation of catalysts used in this invention, there may be preferably used oxides or chlorides, sulfates, nitrates or carbonates which are able to convert to oxides upon strong heating or mixtures thereof. Preparation of catalysts is effected by known methods such as evaporation to dryness, precipitation, mixing of oxides, etc. The catalyst component supported on a carrier may be used. The carrier includes, for example, silica, alumina, silica-alumina, etc.

In practice of this invention, a molecular oxygen-containing gas is added to the starting isobutylene or tert-butanol and gas phase catalytic oxidation thereof is effected in the presence of said catalyst. Molar ratio of isobutylene or tert-butanol : oxygen is preferably 1 : 0.5-3. The feed gas is preferably diluted with an inert gas. The molecular oxygen used for oxidation may be either pure oxygen gas or air, but industrially air is advantageous. Reaction pressure may be from atmospheric pressure to several atms. Reaction temperature is preferably 250-450°C. The reaction may be performed by fluidized bed or fixed bed.

According to the process of this invention, methacrolein and methacrylic acid are industrially and advantageously obtained from isobutylene or tert-butanol. Further, according to this invention, catalysts which are able to produce industrially and advantageously methacrolein and methacrylic acid from isobutylene or tert-butanol are obtained without subjecting Bi compound and W compound to high temperature calcination treatment. Thus, preparation of catalyst is easy.

All "part" in the following examples mean "part by weight" and analysis was carried out by gas chromatography. The conversion of isobutylene or tert-butanol, the selectivities for methacrolein and methacrylic acid produced and per-pass yield are defined as follows:

2

Conversion (%) of isobutylene or tert-butanol

$$= \frac{\text{Mol number of altered isobutylene or tert-butanol}}{\text{Mol number of fed isobutylene or tert-butanol}} \times 100$$

Selectivity (%) for methacrolein

$$= \frac{\text{Mol number of produced methacrolein}}{\text{Mol number of altered isobutylene or tert-butanol}} \times 100$$

Selectivity (%) for methacrylic acid

$$= \frac{\text{Mol number of produced methacrylic acid}}{\text{Mol number of altered isobutylene or tert-butanol}} \times 100$$

Per-pass yield (%) of (methacrolein + methacrylic acid)

$$= \frac{\text{Mol number of produced (methacrolein + methacrylic acid)}}{\text{Mol number of fed isobutylene or tert-butanol}} \times 100$$

Example 1

To 1000 parts of water were added 500 parts of ammonium molybdate, 18.5 parts of ammonium paratungstate, 18.4 parts of cesium nitrate and 354.5 parts of 20 % silica sol, followed by stirring under heating. (Solution A).

Separately, 250 parts of 60 % nitric acid was added to 850 parts of water to obtain homogeneous solution. Then, 57.2 parts of bismuth nitrate was added thereto to dissolve it. To the solution were added successively 238.4 parts of ferric nitrate, 4.7 parts of chromium nitrate, 411.8 parts of nickel nitrate and 60.5 parts of magnesium nitrate to dissolve them. (Solution B).

Solution B was added to Solution A to give slurry and thereto was added 34.4 parts of antimony trioxide, followed by stirring under heating to evaporate most of water.

The resulting cake-like substance was dried at 120°C and then calcined at 500°C for 6 hours and subjected to pressure molding.

Thus obtained catalyst had the following composition:

$Mo_{12}W_{0.3}Bi_{0.5}Fe_{2.5}Sb_1Cr_{0.05}Si_5Ni_6Cs_{0.4}Mg_1O_\ell$

The atomic ratio $\ell$ of oxygen is automatically determined depending on valences of other elements and so will be omitted hereinafter.

This catalyst was charged in a stainless steel reaction tube and a mixed feed gas comprising 5 % of isobutylene, 12 % of oxygen, 10 % of water vapor and 73 % of nitrogen was allowed to pass through the catalyst layer for a contact time of 3.6 seconds and reaction was carried out at 360°C. The results were as follows: conversion of isobutylene: 96 %; selectivity for methacrolein: 86.7 %; selectivity for methacrylic acid: 5.0 %; and per-pass yield of (methacrolein + methacrylic acid): 88.0 %.

Examples 2-9

The following catalysts were prepared according to the procedure of Example 1.

3

Example 2: $Mo_{12}W_{0.5}Bi_{0.3}Fe_{1.5}Sb_{0.8}Cr_{0.05}Si_7Co_7K_{0.3}Zn_1$

Example 3: $Mo_{12}W_{0.3}Bi_1Fe_3Sb_1Cr_{0.1}Si_{10}Ni_4Co_3Rb_{0.5}Mn_{0.5}P_{0.08}$

Example 4: $Mo_{12}W_{0.1}Bi_1Fe_3Sb_1Cr_{0.1}Si_{10}Ni_4Co_3K_{0.2}Cs_{0.3}Cd_{0.5}B_{0.3}$

Example 5: $Mo_{12}W_{0.1}Bi_1Fe_3Sb_1Cr_{0.1}Si_{12}Ni_4Co_3Tl_{0.2}Pb_{0.1}S_{0.8}$

Example 6: $Mo_{12}W_{0.3}Bi_1Fe_3Sb_1Cr_{0.3}Si_{15}Ni_4Co_3Tl_{0.2}Ba_{0.3}Cl_{0.5}$

Example 7: $Mo_{12}W_{0.3}Bi_1Fe_3Sb_1Cr_{0.3}Si_7Ni_4Co_3Tl_{0.2}Mg_{1.5}Zn_{0.5}Ce_{0.5}$

Example 8: $Mo_{12}W_{0.3}Bi_1Fe_3Sb_1Cr_{0.3}Si_{10}Ni_6Tl_{0.2}Mg_{1.5}Sn_{0.5}Ti_{0.5}$

Example 9: $cMo_{12}W_{0.3}Bi_1Fe_3Sb_1Cr_{0.1}Si_{10}Co_5Cs_{0.2}Tl_{0.1}Mg_{0.5}Zn_{1.5}$

Reactions were carried out using these catalysts in the same manner as in Example 1 with changing reaction temperature. The results are shown in Table 1.

Table 1

| | Reaction temperature (°C) | Convertion of isobutylene (%) | Selectivity for methacrolein (%) | Selectivity for methacrylic acid (%) | Per-pass yield of (methacrolein + methacrylic acid) (%) |
|---|---|---|---|---|---|
| Example 2 | 360 | 97 | 87.0 | 3.6 | 87.9 |
| Example 3 | 360 | 95 | 87.9 | 4.5 | 87.8 |
| Example 4 | 365 | 95 | 87.0 | 5.3 | 87.7 |
| Example 5 | 365 | 93 | 91.0 | 3.6 | 88.0 |
| Example 6 | 365 | 94 | 90.0 | 3.7 | 88.1 |
| Example 7 | 360 | 96 | 88.5 | 3.5 | 88.3 |
| Example 8 | 365 | 94 | 88.0 | 5.6 | 88.0 |
| Example 9 | 360 | 96 | 89.0 | 3.0 | 88.3 |

Examples 10-12

Reactions were carried out using the catalysts of Examples 1-3 in the same manner as in Example 1 except that tert-butanol was used as the starting material. The results are shown in Table 2.

Table 2

| | Reaction temperature (°C) | Convertion of tert-butanol (%) | Selectivity for methacrolein (%) | Selectivity for methacrylic acid (%) | Per-pass yield of (methacrolein + methacrylic acid) (%) |
|---|---|---|---|---|---|
| Example 10 | 360 | 100 | 86.0 | 3.3 | 89.3 |
| Example 11 | 360 | 100 | 85.8 | 3.4 | 89.2 |
| Example 12 | 360 | 100 | 85.7 | 3.3 | 89.0 |

Comparative Example 1

A catalyst having the following composition was prepared in the same manner as in Example 1 except that 354.5 parts of 20 % silica sol, 4.7 parts of chromium nitrate and 60.5 parts of magnesium nitrate were omitted.

$Mo_{12}W_{0.3}Bi_{0.5}Fe_{2.5}Sb_1Ni_6Cs_{0.4}$

Reaction was carried out using this catalyst in the same manner as in Example 1. The results were as follows: the conversion of isobutylene: 90 %; the selectivity for methacrolein: 85.0 %; the selectivity for methacrylic acid: 4.3 %; and per-pass yield of (methacrolein + methacrylic acid): 80.4 %.

Comparative Example 2

Reaction was effected using the catalyst of Comparative Example 1 in the same manner as in Example 10. The results were as follows: the conversion of tert-butanol: 100 %; the selectivity for methacrolein: 78.0 %; the selectivity for methacrylic acid: 3.4 %; and per-pass yield of (methacrolein + methacrylic acid): 81.4 %.

Comparative Example 3

A catalyst having the following composition was prepared in the same manner as in Example 1 except that 354.5 parts of 20 % silica sol and 4.7 parts of chromium nitrate were omitted.

$Mo_{12}W_{0.3}Bi_{0.5}Fe_{2.5}Sb_1Ni_6Cs_{0.4}Mg_1$

Reaction was carried out using this catalyst in the same manner as in Example 1. The results were as follows: the conversion of isobutylene: 95 %; the selectivity for methacrolein: 86.0 %; the selectivity for methacrylic acid: 4.8 %; and per-pass yield of (methacrolein + methacrylic acid): 86.3 %.

Comparative Example 4

Reaction was carried out using the catalyst of Comparative Example 3 in the same manner as in Example 10. The results were as follows: the conversion of tert-butanol: 100 %; the selectivity for methacrolein; 84.5 %; the selectivity for methacrylic acid: 3.0 %; and per-pass yield of (methacrolein + methacrylic acid): 87.5 %.

Comparative Example 5

A catalyst having the following composition was prepared in the same manner as in Example 1 except that 4.7 parts of chromium nitrate was omitted.

$Mo_{12}W_{0.3}Bi_{0.5}Fe_{2.5}Sb_1Si_5Ni_6Cs_{0.4}Mg_1$

Reaction was carried out using this catalyst in the same manner as in Example 1. The results were as follows: the conversion of isobutylene: 96 %; the selectivity for methacrolein: 85.3 %, the selectivity for methacrylic acid: 5.0 %; and per-pass yield of (methacrolein + methacrylic acid): 86.7 %.

Comparative Example 6

Reaction was carried out using the catalyst of Comparative Example 5 in the same manner as in Example 10. The results were as follows: the conversion of tert-butanol: 100 %; the selectivity for methacrolein: 84.4 %; the selectivity for methacrylic acid: 3.3 %; and per-pass yield of (methacrolein + methacrylic acid): 87.7 %.

Comparative Example 7

A catalyst having the following composition was prepared in the same manner as in Example 1 except

7

EP 0 267 556 B1

that 18.5 parts of ammonium paratungstate, 354.5 parts of 20 % silica sol and 4.7 parts of chromium nitrate were omitted.

$$Mo_{12}Bi_{0.5}Fe_{2.5}Sb_1 Ni_6 Cs_{0.4}Mg_1$$

Reaction was carried out using this catalyst in the same manner as in Example 1. The results were as follows: the conversion of isobutylene: 90 %; the selectivity for methacrolein: 86.8 %, the selectivity for methacrylic acid: 5.2 %; and per-pass yield of (methacrolein + methacrylic acid): 82.8 %.

Comparative Example 8

Reaction was carried out using the catalyst of Comparative Example 7 in the same manner as in Example 10 to obtain the following results: the conversion of tert-butanol: 100 %; the selectivity for methacrolein: 80.0 %; the selectivity for methacrylic acid: 3.3 %, and per-pass yield of (methacrolein + methacrylic acid): 83.3 %.

## Claims

1. A process for producing methacrolein and methacrylic acid which comprises subjecting isobutylene or tert-butanol to gas phase catalytic oxidation with a molecular oxygen-containing gas in the presence of a catalyst having the composition represented by the following formula:

$$Mo_aW_bBi_cFe_dSb_eCr_fSi_gA_hX_iY_jZ_kO_l$$

wherein Mo, W, Bi, Fe, Sb, Cr, Si and O represent molybdenum, tungsten, bismuth, iron, antimony, chromium, silicon and oxygen, respectively; A represents at least one element selected from the group consisting of nickel and cobalt; X represents at least one element selected from the group consisting of potassium, rubidium, cesium and thallium; Y represents at least one element selected from the group consisting of magnesium, zinc, manganese, cadmium, lead and barium; Z represents at least one element selected from the group consisting of phosphorus, boron, sulfur, chlorine, cerium, tin and titanium; a, b, c, d, e, f, g, h, i, j, k and $l$ each represents atomic ratio of each element; and when a is 12, b is 0.01-2, c is 0.01-3, d is 0.5-4, e is 0.01-3, f is 0.01-3, g is 1-20, h is 1-12, i is 0.01-2, j is 0.01-5 and k is 0-3 and $l$ is the number of oxygen atom necessary to satisfy valence of each of said component.

2. A process according to claim 1 wherein, in the formula, when a is 12, b is 0.03-1, c is 0.1-2, d is 1-3.5, e is 0.1-2, f is 0.03-1, g is 3-18, h is 3-10, i is 0.05-1, j is 0.1-4 and k is 0-1.

3. A process according to claim 1 wherein the starting material is isobutylene.

4. A process according to claim 1 wherein the starting material is tert-butanol.

## Revendications

1. Procédé de préparation de méthacroléine et d'acide méthacrylique, qui consiste à soumettre de l'isobutylène ou du butanol tertiaire à une oxydation catalytique en phase gazeuse, par un gaz contenant de l'oxygène moléculaire en la présence d'un catalyseur ayant la composition représentée par la formule suivante :

$$Mo_aW_bBi_cFe_dSb_eCr_fSi_gA_hX_iY_jZ_kO_l$$

dans laquelle Mo, W, Bi, Fe, Sb, Cr, Si et O représentent le molybdène, le tungstène, le bismuth, le fer, l'antimoine, le chrome, le silicium et l'oxygène, respectivement ; A représente au moins un élément choisi parmi le nickel et le cobalt ; X représente au moins un élément choisi parmi le potassium, le rubidium, le césium et le thallium ; Y représente au moins un élément choisi parmi le magnésium, le zinc, le manganèse, le cadmium, le plomb et le baryum ; Z représente au moins un élément choisi parmi le phosphore, le bore, le soufre, le chlore, le cérium, l'étain et le titane ; a, b, c, d, e, f, g, h, i, j, k et I représentent chacun un rapport atomique de chaque élément ; et quand a est 12, b est compris

8

entre 0,01 et 2, c est compris entre 0,01 et 3, d est compris entre 0,5 et 4, e est compris entre 0,01 et 3, f est compris entre 0,01 et 3, g est compris entre 1 et 20, h est compris entre 1 et 12, i est compris entre 0,01 et 2, j est compris entre 0,01 et 5 et k est compris entre 0 et 3 et 1 est le nombre d'atomes d'oxygène nécessaires pour satisfaire à la valence de chacun des constituants.

2. Procédé suivant la revendication 1, dans lequel, dans la formule, quand a est 12, b est compris entre 0,03 et 1, c est compris entre 0,1 et 2, d est compris entre 1 et 3,5, e est compris entre 0,1 et 2, f est compris entre 0,03 et 1, g est compris entre 3 et 18, h est compris entre 3 et 10, i est compris entre 0,05 et 1, j est compris entre 0,1 et 4 et k est compris entre 0 et 1.

3. Procédé suivant la revendication 1, dans lequel la matière de départ est l'isobutylène.

4. Procédé suivant la revendication 1, dans lequel la matière de départ est le butanol tertiaire.

## Ansprüche

1. Verfahren zur Herstellung von Methacrolein und Methacrylsäure, welches das Umsetzen von Isobutylen oder tert-Butanol in einer katalytischen Gasphasenoxidation mit einem molekularen Sauerstoff enthaltenden Gas in Gegenwart eines Katalysators mit der durch die folgende Formel dargestellten Zusammensetzung umfasst:
$Mo_aW_bBi_cFe_dSb_eCr_fSi_gA_hX_iY_jZ_kO_l$
worin Mo, W, Bi, Fe, Sb, Cr, Si und O Molybdän, Wolfram, Wismut, Eisen, Antimon, Chrom, Silizium und Sauerstoff bezeichnen; A wenigstens ein Element, ausgewählt aus der Gruppe, bestehend aus Nickel und Kobalt, bezeichnet, X wenigstens ein Element, ausgewählt aus der Gruppe, bestehend aus Kalium, Rubidium, Cäsium und Thallium, bezeichnet; Y wenigstens ein Element, ausgewählt aus der Gruppe, bestehend aus Magnesium, Zink, Mangan, Kadmium, Blei und Barium, bezeichnet; Z wenigstens ein Element, ausgewählt aus der Gruppe, bestehend aus Phosphor, Bor, Schwefel, Chlor, Cer, Zinn und Titan, bezeichnet; a, b, c, d, e, f, g, h, i, j, k und l jeweils das Atomverhältnis jedes Elementes bezeichnen; und, wenn a 12 ist, ist b 0,1 bis 2, c 0,01 bis 3, d 0,5 bis 4, e 0,01 bis 3, f 0,01 bis 3, g 1 bis 20, h 1 bis 12, i 0,01 bis 2, j 0,01 bis 5 und k 0 bis 3 und l ist die Anzahl der Sauerstoffatome, die notwendig sind, die Valenzen jeder dieser Komponenten abzusättigen.

2. Verfahren gemäss Anspruch 1, worin in der Formel, wenn a 12 ist, b 0,03 bis 1, c 0,1 bis 2, d 1 bis 3,5, e 0,1 bis 2, f 0,03 bis 1, g 3 bis 18, h 3 bis 10, i 0,05 bis 1, j 0,1 bis 4 und k 0 bis 1 ist.

3. Verfahren gemäss Anspruch 1, worin das Ausgangsmaterial Isobutylen ist.

4. Verfahren gemäss Anspruch 1, worin das Ausgangsmaterial tert-Butanol ist.